(19) Europäisches Patentamt
European Patent Office
Office européen des brevets

(11) EP 3 020 748 A1

# (12) EUROPEAN PATENT APPLICATION

(43) Date of publication:
**18.05.2016 Bulletin 2016/20**

(21) Application number: **14193170.9**

(22) Date of filing: **14.11.2014**

(51) Int Cl.:
*C08G 69/44* (2006.01) *C08L 77/12* (2006.01)
*C07D 413/04* (2006.01) *C07D 413/14* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Maastricht University**
**6211 LK Maastricht (NL)**

(72) Inventors:
- **Noordover, Bart**
  **4811 AN Breda (NL)**
- **Wilsens, Carolus**
  **5653 NC Eindhoven (NL)**
- **Rastogi, Sanjay**
  **5631 LN Eindhoven (NL)**
- **Gubbels, Erik**
  **83308 Trostberg (DE)**

(74) Representative: **CPW GmbH**
**Kasinostraße 19-21**
**42103 Wuppertal (DE)**

## (54) (2-oxazolinyl)-furan based polyamides

(57) The invention pertains to a polyamide obtainable from a mixture comprising a monomer which comprises a (2-oxazolinyl)-furan group, to a resin composition comprising the polyamide, to a shaped product comprising the resin composition and to a process to manufacture a polyamide from a mixture of monomers, whereby a monomer which comprises a 2-oxazolinyl-furan group is reacted with a bis-, tri or multifunctional carboxylic acid, amine, sulfide, alcohol or amide or any combinations thereof.

Fig. 1

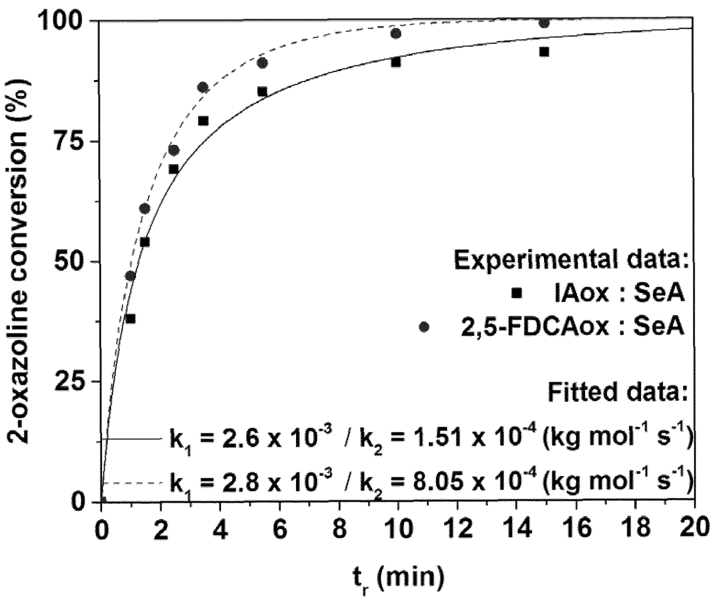




Printed by Jouve, 75001 PARIS (FR)

## Description

The invention pertains to polyamides obtainable from (2-oxazolinyl)-furan derived monomers, to resins and shaped products comprising said polymer, to a process for manufacturing said polyamides and to the use of (2-oxazolinyl)-furan derived monomers for polymerization reactions.

The synthesis, ring-opening, and polymerization of oxazoline groups is known. The interest in oxazoline rings originates from their ability to undergo ring-opening addition reactions with species bearing labile protons, such as carboxylic acids, thiols, phenols, amines, and amides. For this reason, these versatile compounds are used as monomers in addition or step growth reactions such as cationic polymerizations, enzymatic polymerizations or thermally initiated melt polymerizations. Furthermore, bis(2-oxazoline)s have also been used for the chain extension of polymers. Especially thermally initiated melt polymerizations or chain extension reactions are of interest for industrial purposes due to the absence of solvents and solvent recovery systems. Interestingly, besides the synthesis of linear polymer chains, these melt-polymerizations allow for the production of cross-linked polymers in one reaction step.

US 4,474,942 pertains to cross-linked polyesteramides obtained from the polymerization of bis(2-oxazoline) compounds and dicarboxylic acids. The bis(2-oxazoline) compounds comprise an alkyl chain core or an benzene core.

Also Nery et al. (Macromol. Chem. Phys. 2003, 204, p. 1755-1764) describe the polymerization of bis(2-oxazoline) compounds with carboxylic acids. 2,2'-arylene-bis(2-oxazolines) are used as bisoxazoline monomers, as e.g. 2,2'-(1,3-phenylene) bis(2-oxazoline).

However, the rate of branching and crosslinking of such polymers can still be improved. An increased reaction rate is advantageous because it decreases the curing time necessary for producing crosslinked polymers.

Surprisingly, it has now been found that polyamides obtainable from a mixture comprising a monomer which comprises a (2-oxazolinyl)-furan group show increased branching and crosslinking rates and therefore allow faster curing.

The monomer used in the invention is an (2-oxazolinyl)-furan derived monomer which generally is 2,5-furandicarboxylic acid (FDCA)-based. FDCA may be derived from biomass sources and therefore, depending on the choice of the other monomers used in the polymerization, the polyamide of the invention may be based on renewable sources.

The polyamide may be obtained from a (monomer) mixture which comprises a monomer which comprises a (2-oxazolinyl)-furan group (which monomer is herein also referred to as "FDCAox" or "FDCAox monomer") and further comprises a bis-, tri- or multifunctional carboxylic acid, amine, sulfide, alcohol or amide or any combination thereof (also referred to as non-FDCAox monomer).

Preferably, carboxylic acids or alcohols are used, more preferably dicarboxylic acids or diols.

When diols are used, the resulting polymer is a polyetheramide, when dicarboxylic acids are used, a polyesteramide is obtained.

As dicarboxylic acids use can be made of aliphatic dicarboxylic acids, as e.g. malonic acid, sebacic acid, succinic acid, glutaric acid, adipic acid, pimelic acid, suberic acid, azelaic acid, dodecandioic acid, dimer acid, eicosandioic acid and thiodipropionic acid.

Also aromatic dicarboxylic acids may be used as monomers, including but not limited to phthalic acid, naphthalenedicarboxylic acid, diphenylsulfonedicarboxylic acid and diphenylmethanedicarboxylic acid.

In one embodiment of the invention, different (di)carboxylic acids are used in combination.

In the case of diols, aromatic diols are preferred. In one embodiment the aromatic diol has the formula: HO-Ar-(X-Ar)$_m$-OH, wherein Ar is a phenyl or naphthyl group which may be substituted by a methyl or methoxy group, m is 0 or 1, and X is selected from a bond, S, O, SO, $SO_2$, CO, $CH_2$ and $C(CH_3)_2$. Positioning of the hydroxy groups is preferably para with respect to other functional groups, but may also be *meta* or *ortho.*

Examples are aromatic diols of the formulae:

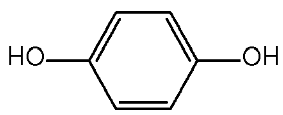

Hydroquinone

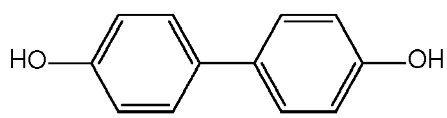

Biphenol

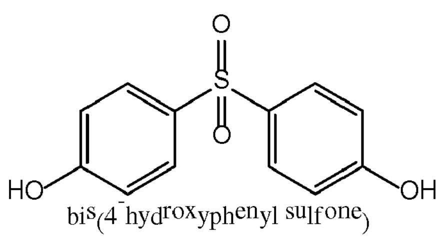

bis(4-hydroxyphenyl sulfone)

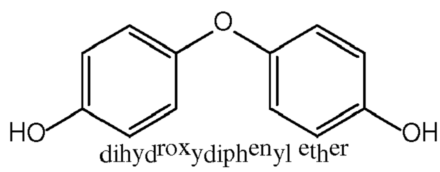

dihydroxydiphenyl ether

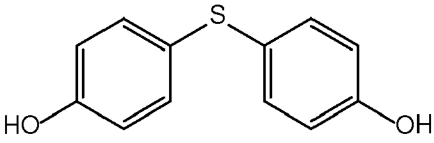

dihydroxydiphenyl sulfide

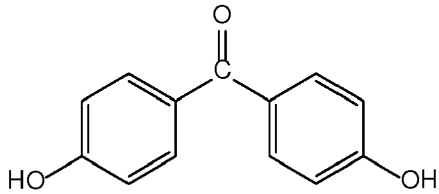

dihydroxybenzophenone

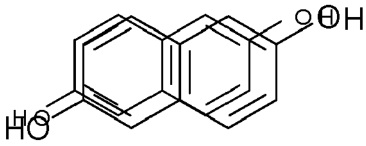

dihydroxynaphthalene

The aromatic phenyl or naphthyl groups may independently be substituted by one to four methyl or methoxy groups. The positions of the hydroxy groups may be *meta* or *ortho,* but the para position as depicted in the above examples is preferred.

In one embodiment the FDCAox monomer used in the current invention is of formula I,

I

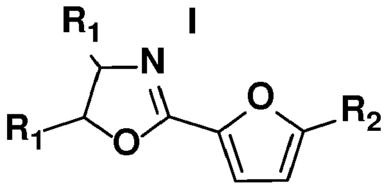

where each $R_1$ is independently of one another selected from a proton and an alkyl group and $R_2$ is selected from moieties comprising an oxazolinyl, carboxylic acid, amine, amide or sulfide group.

The FDCAox monomer of formula I comprises moiety $R_2$. Moiety $R_2$ comprises one of the above listed functional groups. Moiety $R_2$ may also comprise a linker group, e.g. a (bivalent) carbon group as linker between the functional group comprised in $R_2$ and the furan ring of the FDCAox monomer. The linker may be any suitable group, including a furan group, a benzene group, an alkanediyl group or combinations thereof.

In a preferred embodiment, the FDCAox monomer used in the invention is a (substituted) furan-based bis(2-oxazoline) of formula II or III:

II

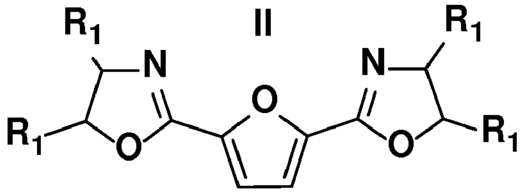

III

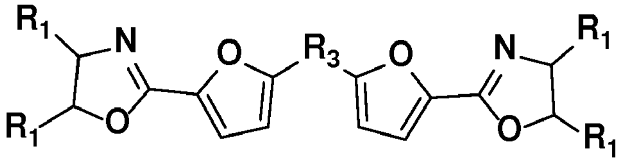

where each $R_1$ is independently of one another selected from a proton and an alkyl group and where $R_3$ is selected from a C=O group and an alkanediyl group.

In one embodiment a FDCAox monomer of formula II is used, more particular 2,5-bis(4,5-dihydrooxazol-2-yl)furan, (referred to as "2,5-FDCAox", where each R1 is a proton). When this monomer is reacted with a dicarboxylic acid a polyesteramide of the following structure is obtained:

-(furan-C(O)NHC$H_2$C$H_2$OC(O)R'C(O)OC$H_2$C$H_2$NHCO$)_n$-, wherein R' is a bivalent hydrocarbon radical originating from the core of the dicarboxylic acid.

An alkyl is an aliphatic moiety with a total number of carbon atoms between 1 and 10, preferably between 1 and 5, i.e. a functional group comprising only carbon and hydrogen atoms derived from an alkane by removing one hydrogen atom. This definition includes unbranched carbon chains and branched carbon chains, i.e. isomers.

Examples are: methyl, ethyl, propyl or isopropyl.

An alkanediyl group is of the formula $-(CH_2)_n-$, where n is between 1 and 12, preferably between 1 and 5.

The monomer mixture from which the polyamide of the invention is obtainable may comprise a FDCAox monomer or combinations of different FDCAox monomers together with a non-FDCAox monomer or combinations of different non-FDCAox monomers. In such an embodiment, the above listed embodiments of FDCAox monomers and non-FDCAox monomers may be used.

The equivalent ratio between FDCAox and non-FDCAox monomer in the monomer mixture may be 1:1 to 100:1, preferably 1-5:1, more preferably 1.5-2.5:1. Specific embodiments are equivalent ratios of 2.25:1, 2:1, 1.75:1 and 1.25:1.

The polyamide of the present invention has a crosslinking degree of at least 5%, preferably at least 10 %, more preferably at least 20% or even higher.

During polymerization of the polyamide of present invention the branching and crosslinking reactions occur more rapidly, and hence shorter curing times can be realized during processing of the polyamide.

Without being bound by theory the enhanced branching and cross-linking might be attributed to an increased nucleophilicity of the 2,5-furandicarboxamide. The 2,5-furandicarboxamide is formed when the 2-oxazoline ring of the FDCAox monomer is opened during the polymerization reaction. The increased nucleophilicity could result from intra-molecular hydrogen bonding between the free electron pairs of the oxygen of the furan ring with the proton of the amide group. As a result, the N-H bond of the amide groups weakens and the nitrogen becomes more electron rich and thus a better nucleophile. This might subsequently enhance the reactivity of this amide group towards (2-oxazoline) groups. A tertiary amide and a trifurcate bond could be formed which results in branching.

A potential reaction scheme of a monomer as used in the invention with a dicarboxylic acid is shown below:

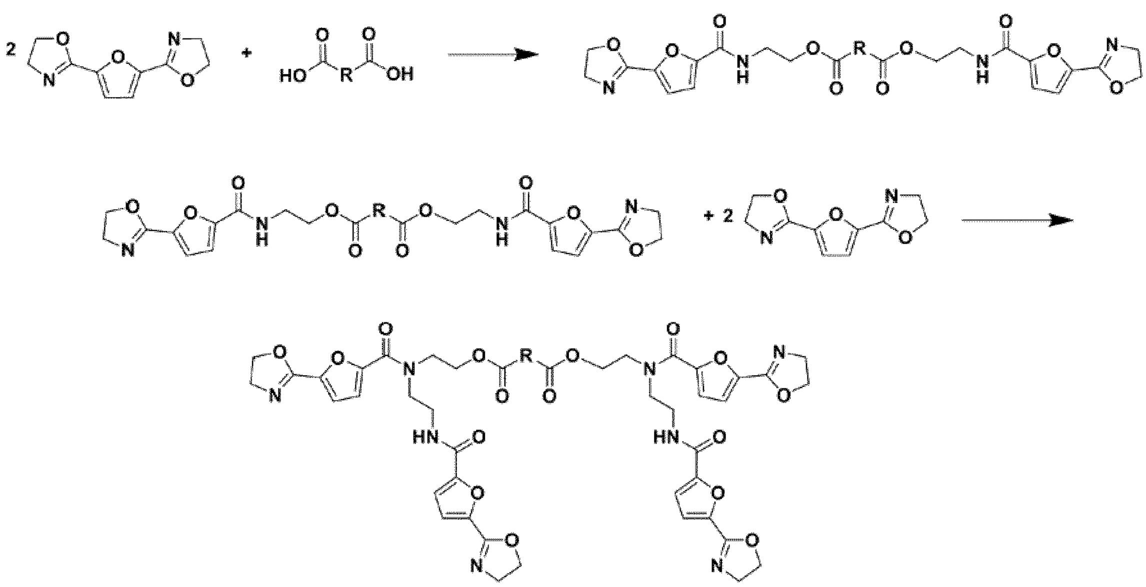

The present invention also relates to a resin composition comprising the polyamide of the current invention.

In one embodiment the resin is a thermoset resin.

The resin composition may also be a mixture of different polyamides of this invention. For example, a polymer derived from a monomer mixture with a ratio of the FDCAox monomer used in the invention to the non-FDCAox monomers of 2:1 may be mixed with a polymer derived from a monomer mixture with a ratio of the FDCAox monomer to the non-FDCAox monomers of 1:2. Alternatively or in addition to this embodiment, the polymers of the resin composition may be derived from different monomer mixtures comprising different FDCAox and non-FDCAox monomers in combination.

For example, the resin composition may comprise at least two different polyesteramides derived from monomer mixtures comprising different dicarboxylic acids and/or different FDCAox monomers, optionally from monomer mixtures of different equivalent ratios of the dicarboxylic acid to the FDCAox monomers.

In one embodiment the resin comprises reinforcements and/or fillers.

Reinforcements may be fibrous reinforcements, preferably inorganic, natural or synthetic fibers. Suited are e.g. glass fibers, carbon fibers, polyamide fibers, aramid fibers, nylon or polyester fibers. In one embodiment different fibrous reinforcements are combined in one resin composition.

In combination with such fibrous reinforcements or alone, filler materials may be added to the resin composition. Examples of such fillers include oxides, hydroxides, carbonates, silicates, particulate carbon or metal powder.

Fibrous reinforcements and fillers may each be added to the resin composition in an amount of 10 wt% - 70 wt%, more preferably 20 wt% to 80 wt%, and more preferably 30 to 90 wt%.

The resin composition of the invention may be used to form shaped products. Shaped products comprising the resin composition of current invention are a further object of this invention.

The shaped products may be films, polymer glasses, coatings, adhesives or laminates.

The shaped products may be obtained by polymerization of the FDCAox monomer with non-FDCAox monomers.

Another object of current invention is a process for manufacturing a polyamide from a mixture of monomers, whereby a monomer which comprises a 2-oxazolinyl-furan group is reacted with a bis-, tris- or multifunctional carboxylic acid, amine, sulfide or an alcohol or any combinations thereof.

The embodiments of the monomer which comprises a 2-oxazolinyl-furan group are described above. Preferably, a monomer of formula I as defined in claim 3 and above, more preferably a monomer of formula II or III as defined in claim 4 and above is used for the polymerization reaction.

In a preferred embodiment 0.01-10 wt%, preferably 0.1-7 wt%, more preferably 0.2 -5 wt%, or 0.5 -2 wt% of a catalyst is added to the mixture of monomers.

The catalyst may be selected from phosphorous acid esters, organic phosphonous acids and inorganic salts or combinations thereof. Preferably, phosphorous acid esters are employed, as e.g. diesters and triesters, in particular triphenyl phosphite (TPP), tris(nonylphenyl) phosphite, triethyl phosphite, tri-n-butyl phosphite, tris(2-ethylhexyl) phosphite, tristearyl phosphite, diphenylmonodecyl phosphite, tetraphenyl dipropylene glycol diphosphite, tetraphenyltetra(tridecyl)pentaerythritol tetraphosphite, diphenyl phosphite, 4,4'-butylidenebis(3-methyl-6-t-butylphenyl-ditridecyl) phosphite and bisphenol-A pentaerythritol phosphite. Two or more of these catalysts may be used.

For the manufacturing process a combination of different monomers may be used. One or more different FDCAox monomers may be mixed with one or more bis-, tri-or multifunctional carboxylic acid, amine, sulfide, alcohol or amide (the non-FDCAox monomers).

Usually, the reaction temperature will be at least 150 °C, preferably at least 180 °C, more preferably at least 200 °C. As a general maximum for the reaction temperature 350 °C may be mentioned.

The polymer branching may be influenced by adjusting the reaction temperature and the catalyst concentration. With increasing amounts of catalyst (the branching degree of the polymer increases with increasing catalyst concentration. For example this can be observed when 0, 0.5, 1, 2, 5 or 7 wt% of catalyst, as e.g. triphenyl phosphite, are added to a monomer mixture comprising FDCAox (e.g. 2,5-FDCAox and a bifunctional carboxylix acid (e.g. sebacic acid) at an equivalent ratio of 1-2.5 eq. of FDCAox to 1 of the bifunctional carboxylic acid and using a reaction temperature of 200°C). Similarly, the branching frequency increases when the reaction temperature is increased, independently of the addition of catalyst. For example, when the reaction temperature of the monomer mixture (with or without catalyst) is chosen from 150°C, 170°C, 200°C, 210°C and 230°C, each further increase of the reaction temperatures will give accelerated branching, meaning that the branching concentration after the same reaction time is higher in samples with a higher reaction temperature.

In general, melt polymerization procedures may be used to manufacture the polyamide of the invention, alternatively the polyamide of the invention may be manufactured in a high boiling solvents, including but not limited to N,N'-dimethylformamide, N,N'dimethylacetamide, N-Methyl-2-pyrrolidone (NMP) or dimethylsulfoxide.

In one embodiment solvents are added to the polymerization reaction, preferably dipolar solvents, as e.g. NMP (*N*-Methyl-2-pyrrolidone), and are deposited on a surface prior to polymerization. During this polymerization process (which is sometimes referred to as curing), the solvent evaporates, leaving a thin cross-linked coating on the substrate.

A nitrogen-rich atmosphere is preferred as a reaction condition.

The reaction temperature may be kept constant during the reaction but it may also be increased during the curing process. Especially for curing reactions it might be advantageous to increase the reaction temperature during the reaction. For example, a temperature of 150°C could be used at the reaction start which is gradually or step-wise increased to the maximum reaction temperature.

For the polymerization reaction the (combinations of) FDCAox and the (combinations of) non-FDCAox monomers may be mixed at an equivalent ratio of 1:1 to 100:1, preferably 1-5:1, more preferably 1.5-2.5:1. Specific embodiments are equivalent ratios of 2.25:1, 2:1, 1.75:1 and 1.25:1.

The present invention is further directed to the use of monomers comprising a (2-oxazolinyl)-furan group for polymerization reactions. The embodiments of the monomer which comprises a (2-oxazolinyl)-furan group are described above. Preferably, a monomer of formula I as defined in claim 3 and above, more preferably a monomer of formula II or III as defined in claim 4 and above is used for the polymerization reaction.

The invention is further illustrated by the following non-limiting examples.

1. Synthesis of compounds

2,5-Furandicarboxylic acid (2,5-FDCA) was obtained from Atomole, China (>99 wt%). Sebacic acid (SeA), 2-chloroethylamine hydrochloride, triphenylphosphite (TPP), thionyl chloride, sodium hydroxide, and potassium hydroxide were purchased from Sigma. Irganox 1330 antioxidant was a kind gift from Ciba Specialty Chemicals. 1,3-bis(4,5-dihydrooxazol-2-yl)benzene (IAox) was purchased from TCI Europe. Deuterated dimethyl sulfoxide (DMSO-*d*6, 99.9% atom D) was acquired from Cambridge Isotope Laboratories. Methanol, dichloromethane (dried over $Al_2O_3$), N,N-dimethylformamide (DMF) and N-methyl-2-pyrrolidone (NMP) were obtained from Biosolve. All chemicals were used as received, unless stated otherwise.

a) Preparation of N,N'-bis(2-chloroethyl)furan-2,5-dicarboxamide

2,5-furandicarboxylic acid (16.4 g, 0.105 mol) was reacted with an excess of thionyl chloride (27.5 mL, 0.23 mol), in the presence of a catalytic amount of DMF (100 $\mu$L) in a 250 mL three-neck flask at 80 °C for four hours. A condenser was connected to the flask to allow refluxing of thionyl chloride and the reaction was conducted under an argon rich atmosphere. The released gaseous $SO_2$ and HCl were passed through an aqueous NaOH solution, neutralizing the hydrochloric acid. After 4 hours of reaction time, the mixture was cooled with an ice-bath followed by the application of reduced pressure for 30 minutes to remove any residual thionyl chloride. The obtained 2,5-furandicarboxylic acid chloride was dissolved in anhydrous dichloromethane (150 mL) and dropwise added to an aqueous solution of 2-chloroethylamine hydrochloride (24.4 g, 0.21 mol) and NaOH (16.4 g, 0.41 mol) under vigorous stirring. After 2 hours of reaction time, the organic phase was distilled off using reduced pressure and the water phase was filtered. The product, N,N'-bis(2-chloroethyl)furan-2,5-dicarboxamide, was obtained as white powder and was dried overnight under reduced pressure at 40 °C. Yield = 25.3 g (86.4%). $^1$H NMR (400 MHz, DMSO-*d*6, $\delta$, ppm): 7.19 (s, 2H, furan), 3.75 (t, 4H, $ClCH_2$), 3.60 (d, 4H, $NCH_2$).

b) Preparation of 2,5-bis(4,5-dihydrooxazol-2-yl)furan (2,5-FDCAox)

N,N'-bis(2-chloroethyl)furan-2,5-dicarboxamide (15.00 g, 0.054mol) and NaOH (4.8 g, 0.12 mol) were dissolved in 50 mL methanol in a 250 mL round-bottom flask, and were allowed to reflux for 4 hours. During the reaction white crystals precipitated. The formed product was isolated using filtration and washed with water prior to drying *in vacuo* overnight at 40 °C. Yield = 8.98 g (81.1%). $^1$H NMR (400 MHz, DMSO-*d*6, $\delta$, ppm): 7.13 (s, 2H, furan), 4.35 (t, 4H, $NCH_2$), 3.92 (t, 4H, $OCH_2$). $^{13}$C NMR (400 MHz, DMSO-*d*6, $\delta$, ppm): 155.4 (O-C=N), 144.8 (O-C=C), 115.9 (C-C=C), 68.0 ($CH_2$-O), 55.0 ($CH_2$-N). Elemental analysis for $C_{10}H_{10}N_2O_3$, observed (calculated): C = 58.25% (58.19%), H = 4.89% (4.82%), and N = 13.59% (13.58%).

c) General melt polymerization procedure.

Reaction mixtures were prepared by weighing and grinding 2,5-FDCAox (1.0 to 2.5 eq), SeA (1 eq), Irganox 1330 (1 wt%), and TPP (0 to 7 wt%) using a mortar and pestle. Samples prepared for DMAc-SEC and DSC analysis were loaded into 1 mL glass vials and immersed in an oil-bath at the desired temperature of 160 °C for the desired reaction time (0 - 60 minutes) prior to analysis. Samples prepared for DMTA analysis were preheated to 160 °C for one minute until a clear casting liquid was obtained. The casting liquid was then poured onto a Teflon film and cured in an oven at 200 °C under a nitrogen rich atmosphere for 10 minutes to one hour.

d) Preparation of solvent-borne coatings.

2,5-FDCAox (2.25 eq) was cured with SeA (1 eq) in the presence of 5 wt% TPP using the following procedure. A solution of 2,5-FDCAox (139.3 mg, 0.675 mmol), sebacic acid (60.7 mg, 0.3 mmol) and triphenylphosphite (10 mg, 5 wt%) was prepared in 0.6 mL N-Methyl-2-Pyrrolidone at 100 °C. This solution was subsequently applied to Q-panels, preheated in an oven at 200 °C, using a coating applicator frame with a spacing of 120 $\mu$m. The obtained coatings were cured by heating the coated panels in an oven at 200 °C for 1 hour under a nitrogen rich atmosphere.

2. Characterization methods

The reaction kinetics involved during the polymerization of bis(2-oxazoline)s can be described using differential equations **(1) - (3).** To obtain these differential equations, it is assumed that both reactions are irreversible and second order, that the 2-oxazoline groups are equireactive, and that the secondary amides are equireactive. In differential equations **(1) - (3),** the unreacted (2-oxazoline) bulk concentration is denoted as [OX] in mol/kg. Similarly, the bulk concentrations of the formed ester groups, secondary amide groups, and tertiary amide groups are denoted as [Ester], [Sec. Amide], and [Tert. Amide] respectively in mol/kg.

$$\textbf{(1)} \quad \frac{\delta[\mathrm{Acid}]}{\delta t} = -\frac{\delta[\mathrm{Ester}]}{\delta t} = -\frac{\delta[\mathrm{Sec.Amide}]}{\delta t} = -k_1\,[\mathrm{Acid}][\mathrm{OX}]$$

$$\textbf{(2)} \quad \frac{\delta[\mathrm{OX}]}{\delta t} = -k_1\,[\mathrm{Acid}][\mathrm{OX}] - k_2[\mathrm{Sec.\,Amide}][\mathrm{OX}]$$

$$\textbf{(3)} \quad \frac{\delta[\mathrm{Tert.Amide}]}{\delta t} = k_2\,[\mathrm{Sec.\,Amide}][\mathrm{OX}]$$

**$^1$H NMR and $^{13}$C NMR spectroscopy** was performed on a 400 MHz Brucker AVANCE-III spectrometer. Samples were prepared by dissolving 5-10 mg of monomer or polymer in 0.5 - 1 mL deuterated dimethyl sulfoxide (DMSO-d6) and were referenced against tetramethylsilane (TMS).

**N,N-Dimethylacetamide size exclusion chromatography (DMAc-SEC) was** performed on a Waters Alliance system equipped with a Water 2413 refractive index detector (40 °C), a Waters 2695 separation module, a Waters 2996 photodiode array detector, a PSS GRAM guard column followed by 2 PSS GRAM columns in series of 100 Å (10 μm particles) and 3000 Å (10 μm particles) at 60 °C. DMAc was used at a flow rate of 1 mL/min and molecular weights were calculated against polystyrene standards (Polymer Laboratories, $M_p$ = 580 Da up to $M_p$ = 7.1 × $10^6$ Da). Polymer samples prepared in a concentration of 5 mg / mL and were filtered through a 0.2 μm PTFE filter (13 mm, PP housing, Alltech) prior to injection.

**Thermal stability of the materials** synthesized in this study was evaluated using thermogravimetricanalysis (TGA). Experiments were performed on a TA Instruments TGA Q500 in a nitrogen rich atmosphere. Samples were heated from 20 to 800 °C, at a heating rate of 10 °C/min.

**Differential scanning calorimetry (DSC)** was performed using a TA Instruments DSC Q100. Two heating and cooling runs were performed -50 °C to 220 °C at 10 °C/min and the glass transition temperatures were determined from the second heating run.

**Dynamic mechanical thermal analysis (DMTA)** was performed using a TA Instruments Q800. Samples were prepared by mixing and grinding the desired ratio of 2,5-FDCAox to SeA powder prior to the addition of 5 wt% TPP and 1 wt% Irganox 1330. The mixtures were heated to 160 °C until a clear casting liquid was obtained (generally 30 seconds to 1 minute under mild stirring). The obtained liquid was poured on a Teflon film and cured in an oven at 200 °C for 10 minutes to one hour under a nitrogen atmosphere. The obtained polymer films were reshaped into rectangles of 20 mm x 5 mm and were measured in the DMTA from -50 °C to 225 °C at a heating rate of 2 °C/min, a frequency of 1 Hz, and an amplitude of 10 μm. If required, the samples were kept under isothermal conditions at 200 °C to follow the curing process of the sample. Glass transition temperatures were calculated from the maximum in the tan δ trace obtained during heating of the sample.

**Coating properties**

Coating thicknesses of the solvent-borne coatings were measured using a magnetic induction coating thickness gauche (LD0400 by Thermimport Quality Control). Reverse impact tests were performed by dropping a weight of 1 kg from a controlled height of 100 cm on the backside of the coated panels and the pencil hardness test was performed by scratching the obtained coating with pencils of increasing hardness. Chemical resistance was determined by the acetone double rub test in which the material is rubbed back and forth (double rubs) by an acetone drenched cloth 100 times.

Example 1 - Comparison of reaction rate

A polyesteramide according to the invention was prepared as described above from a mixture of monomers comprising 2,5-bis(4,5-dihydrooxazol-2-yl)furan (2,5-FDCAox) and sebacic acid. As a comparison experiment a polyesteramide was prepared in the same way from a mixture of monomers comprising 1,3-bis(4,5-dihydrooxazol-2-yl)benzene (IAox) and sebacic acid. IAox is a bisoxazoline compound having a benzene core instead of a furan ring.

To study the rates of the reactions occurring during polymerization, the 2-oxazoline conversion was monitored during the polymerization of an equimolar mixture of 2,5-FDCAox or IAox and sebacic acid (SeA) at 160 °C. The monomers were mixed in the solid state and heated for a fixed reaction time ($t_r$) using a salt bath. After cooling back to room temperature, the obtained polymers were dissolved in deuterated dimethylsulfoxide (DMSO-$d_6$) and analyzed using $^1$H NMR analysis.

The 2-oxazoline conversion was calculated from the $^1$H NMR spectra of the products obtained after polymerization for varying times $t_r$ (1 to 15 minutes). First, the fraction of unreacted 2-oxazoline groups was obtained after dividing the value of the integral of the $CH_2N$ resonance (4.35 ppm) by the value of the integral of the resonance of the furanic protons (7.13 ppm). Next, the conversion was calculated via subtraction of the 2-oxazoline fraction from 1.00 and converted to percentages. A correction for the number of protons was performed prior to the subtraction. Similarly, the fraction of unreacted 2-oxazoline groups in the IAox polymerization was calculated using the resonance of $CH_2N$ proton at 4.35 ppm and the aromatic proton at 8.30 ppm. The 2-oxazoline conversion as a function of reaction time was used for data fitting with differential equations **(1)** - **(3).** The data fitting was performed using non-linear regression, yielding reaction constants $k_1$ and $k_2$ (Figure 1).

From the experimental data shown in Figure 1 it can be clearly seen that the 2,5-FDCAox monomer is consumed faster during polymerization with SeA than the IAox monomer. Interestingly, the observed $k_1$ reaction constant found after data fitting for the polymerizaton of the system containing IAox ($k_1$ = 2.6 x $10^{-3}$ kg $mol^{-1}$ $s^{-1}$) and 2,5-FDCAox ($k_1$ = 2.8 x $10^{-3}$ kg $mol^{-1}$ $s^{-1}$) are comparable, suggesting that both oxazoline-functional monomers have a comparable reactivity towards carboxylic acid groups. Furthermore, from Figure 1 is observed that the $k_2$ reaction constant observed in the polymerization of 2,5-FDCAox with SeA is roughly 5 times larger (8.05 x $10^{-4}$ kg $mol^{-1}$ $s^{-1}$) than the $k_2$ reaction constant found in the polymerization of IAox with SeA (1.51 x $10^{-4}$ kg $mol^{-1}$ $s^{-1}$).

The results obtained in the $^1$H NMR analysis suggest that branching occurs at an enhanced rate in the polymerization of 2,5-FDCAox with SeA compared to the polymerization of IAox with SeA.

Example 2 - Effect of branching of $M_w$ and PDI

To investigate the effect of this branching reaction on the $M_w$ (molecular weight) and PDI (polydispersity index) of the polymers, DMAc-SEC was performed on the materials obtained after polymerization of equimolar mixtures of 2,5-FDCAox with SeA and IAox with SeA at 160 °C for $t_r$ varying from 1 to 60 minutes. Figure 2a shows an overview of the $M_w$ and PDI obtained for the 2,5-FDCAox and IAox based systems. Figure 2b and 2c show the SEC chromatograms obtained as a function of reaction time for the 2,5-FDCAox and IAox based systems, respectively.

From Figure 2a, it can be seen that the $M_w$ and PDI increase faster during the polymerization of 2,5-FDCAox with SeA compared to the polymerization of IAox with SeA. For example, a $M_w$ of 33.2 kg/mol and a PDI of 4.8 is achieved in the polymerization of 2,5-FDCAox with SeA after 15 minutes of reaction time. In contrast, the isophthalic acid (IA) based polymer has only reached a $M_w$ 20.4 kg/mol and a PDI of 3.1 after 15 minutes of polymerization. Such a difference in molecular weight corresponds well to the difference in 2-oxazoline consumption observed in the $^1$H NMR analysis.

From Figure 2b, it can be seen that branching of the polymer chains is occurring in the polymerization of 2,5-FDCAox with SeA, as is detected by the elution of a high molecular weight fraction in the DMAc-SEC chromatogram for polymers having a $t_r$ of 10 minutes or longer. This high molecular weight fraction, characteristic for branched chains formed through chain coupling, is indicated by the black arrow in Figure 2b. Interestingly, the continued branching occurring during polymerization results in the formation of a cross-linked and insoluble polymer after 25 minutes of reaction time. In contrast, the polymer obtained after 60 minutes of polymerization of IAox with Sea is fully soluble in the DMAc solvent, indicating that the polymer is not cross-linked.

These observations show that branching occur during a polymerization of IAox with SeA at a lower rate than in polymerizations involving the 2,5-FDCAox monomer of the invention.

Example 3 - Thermal properties of poly(ester amide)s

The thermal properties of the polymers obtained after one hour of polymerization were evaluated using DSC analysis. The samples were measured at a heating and cooling rate of 10 °C / min and the second heating runs are depicted in Figure 3. It is observed that the glass transition temperature ($T_g$) of the 2,5-FDCAox based polymer is roughly 5 °C higher than the $T_g$ of the IA based polymer. This difference in $T_g$ is attributed to the cross-linked nature of the 2,5-FDCAox based polymer, as is confirmed by the broad temperature range of the glass transition. Furthermore, the 2,5-FDCAox based polymer shows no melting transitions prior to degradation. It should be noted that, even though 2,5-FDCA based polyamides generally do not exhibit crystallization or melting, the cross-linked nature of the 2,5-FDCA based poly(ester amide) synthesized in this study is expected to limit crystallization and melting even further. In contrast, the IAox based polymer shows a peak melting temperature at 105 °C, which is in good agreement with the melting range between 110 to 115 °C reported in literature.

Example 4 - Thermal properties of the cured thermosets

The addition of TPP can be used to selectively accelerate the branching reaction occurring in the polymerization of 2,5-FDCAox with SeA. The addition of this catalyst decreases the reaction and curing time, facilitating the synthesis of renewable polymer glasses and coatings within reasonable reaction times. In order to investigate the thermal properties of the polymer synthesized via this route, DMTA analysis was performed on cured glasses and the glass transition temperatures were determined from the peak in the tan($\delta$) trace. Figure 4 shows the characteristic behavior of systems of a) IAox : SeA (2.25 : 1) and b) 2,5-FDCAox : SeA (2.25 : 1) containing 5 wt% TPP. The samples were cured for 10 minutes in an oven preheated to 200 °C prior to the DMTA analysis to prevent breaking of the samples above $T_g$ during the DMTA experiment.

From the data shown in Figure 4, it can be clearly seen that the IAox based polymer obtained after 10 minutes of curing is slightly cross-linked, as is indicated by the low storage modulus (E') of 0.53 MPa above the $T_g$ (59.6 °C). Such a low storage modulus above $T_g$ corresponds to a high molecular weight between entanglements and indicates that the system is not densely cross-linked. As expected, the curing process continues after heating to temperature of 120 °C and higher at a heating rate of 2 °C, as is indicated by the gradual increase of both storage and loss moduli. No changes in storage and loss modulus are observed after 30 minutes of curing (thus after 90 minutes of the DMTA experiment), indicating that the cross-linking reaction is complete.

Interestingly, the DMTA analysis of the polymer obtained after heating, casting and curing the 2,5-FDCAox and SeA mixture (ratio = 2.25 : 1) for 10 minutes in the presence of 5 wt% TPP shows a $T_g$ at 143.2 °C and a plateau modulus (E') of 22.01 MPa. This increased glass transition temperature, compared to the glass transition temperature of the IAox based polymer obtained after 10 minutes of curing, indicates that the 2,5-FDCAox based polymer is more densely cross-linked after 10 minutes of reaction time. Furthermore, no significant increase of the plateau modulus is observed upon further heating to temperatures above the $T_g$ followed by curing under isothermal conditions for 60 minutes at 200 °C.

For both systems, obtained after 60 minutes of curing at 200 °C, a second heating run was performed at 2 °C/min to determine the $T_g$ of the fully cross-linked polymers. Interestingly, the $T_g$ of the IAox based polymer (155.1 °C) was comparable with the $T_g$ of the 2,5-FDCAox based polymer (156.3 °C), indicating that the fully cross-linked materials have comparable glass transition temperatures. The DMTA experiments indicate that the cross-linking in the 2,5-FDCAox polymerization with SeA in the presence of TPP proceeds significantly faster than for the IAox based system. Although the exact mechanism of the TPP catalysis is unknown, it can be concluded that the application of 2,5-FDCAox in polymerization in the presence of TPP drastically reduces the curing times compared to similar polymerizations containing IAox as bis(2-oxazoline) monomer. To see if one can correlate the normalized intensity and the concentration of tertiary amide groups to the glass transition temperature, DMTA experiments were performed on polymers with varying 2,5-FDCAox to SeA ratios. The polymers were cured for 60 minutes at 200 °C prior to the DMTA analysis to ensure that the samples were fully cross-linked. Furthermore, FTIR analysis was performed on different spots of the samples obtained after DMTA analysis and the normalized intensity of the vibration band at 1417 $cm^{-1}$ was calculated following the FTIR analysis procedure. Figure 5 shows the normalized intensity signal obtained from FTIR plotted against the glass transition temperatures found via DMTA analysis for the different polymer glasses.

It can be seen that the normalized intensity varied slightly over the surface of the cured samples, indicating that the obtained thermosets are probably not homogeneously cured. Nonetheless, it is clearly visible that the normalized intensities obtained from FTIR analysis increase linearly with the increasing $T_g$ of the polymers. These data suggests that the increase of the glass transition temperature is directly correlated to the increase of the bulk concentration of tertiary amides and thus to the concentration of branches and cross-links.

Example 5 - Coating performance of solvent-borne coatings

Solvent-borne coatings were prepared using NMP as a solvent and were cured at 200 °C, as is described above in the coating procedure. The obtained coatings had a thickness of approximately 35 $\mu$m and were sufficiently cross-linked as was indicated by the acetone double rub test. Furthermore, the coatings were soft, as became evident from the pencil hardness test (2H). This indicates that the materials were not densely cross-linked, which was confirmed through FTIR analysis; the coatings showed an average normalized intensity of 1.6 (the vibration at 1417 $cm^{-1}$) corresponding to a $T_g$ around 80 °C, as follows from the data shown in Figure 5. This implies that the presence of solvent

during the curing process limits the cross-link density, since the bulk cured samples exhibited significantly higher $T_g$ in the range between 140-160 °C. Although the coatings were not densely cross-linked, they showed ductile deformation and showed no crazing or cracking during rapid deformation in reverse impact testing. These coating results indicate that these 2,5-FDCA based poly(ester amide)s are good candidates for application in both thermally cured and solvent-borne polymer glasses and coatings.

Figure 1. Conversion of 2-oxazoline groups over time during melt-polymerization of equimolar mixtures IAox and SeA (squares) or 2,5-FDCAox and SeA (circles) at 160 °C, as calculated from the $^1$H NMR spectra. The theoretical curves and reaction constants found after data fitting are shown for the IAox system (continuous line) 2,5-FDCAox system (dotted line).

Figure 2. Changes of the weight-average molecular weight ($M_w$) and over time during the polymerization of 2,5-FDCAox with SeA and IAox with SeA in an equimolar ratio performed at 160 °C, as observed in DMAc-SEC analysis. Figure 2a shows an overview of changes in the $M_w$ and of both reactions over time, whereas Figures 2b and 2c show changes in the DMAc-SEC chromatograms observed over time for the systems containing 2,5-FDCAox and IAox respectively. The dotted lines in Figure 2a are added to guide the eye.

Figure 3. Second heating DSC curves of the polymers obtained after one hour of polymerization of 2,5-FDCAox and SeA and and IAox and SeA at 160 °C. It can be seen that the system containing IAox has a lower glass transition temperature and shows a small melting endotherm above 100°C. In contrast, the 2,5-FDCAox based polymer does not show a melting endotherm and exhibits a broad glass transition temperature, characteristic for branched and cross-linked polymers.

Figure 4. Variation of the temperature, tan(δ), E', and E" during a heating (2 °C / min) and isothermal curing (200 °C, 60 min) as observed in the DMTA analysis in TPP (5 wt%) catalyzed systems of a) IAox : SeA (ratio = 2.25 : 1) and b) 2,5-FDCAox : SeA (ratio = 2.25 : 1). *N.B.* both systems were heated until a clear casting liquid was obtained, which was poured on a Teflon film and cured at 200 °C for 10 minutes prior to DMTA analysis.

**Figure 5.** $T_g$ values (°C) calculated from the maximum in the tan(δ) trace obtained from a DMTA heating run performed at 2 °C/min versus the normalized intensity of the vibration band at 1417 $cm^{-1}$ obtained from FTIR analysis of cured systems having varying 2,5-FDCAox to SeA ratio's.

## Claims

1. A polyamide obtainable from a mixture comprising a monomer which comprises a (2-oxazolinyl)-furan group.

2. The polyamide of claim 1 where the mixture further comprises a bis-, tri- or multifunctional carboxylic acid, amine, sulfide, alcohol or amide, or any combination thereof.

3. The polyamide of claim 1 or 2 where the monomer comprising the 2-oxazolinyl-furan group is of formula I :

I

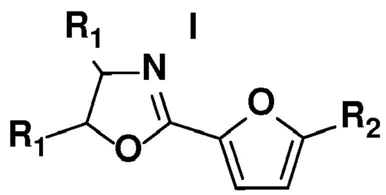

where each $R_1$ is independently of one another selected from a proton and an alkyl group, and $R_2$ is selected from moieties comprising an oxazolinyl, carboxylic acid, amine, amide or sulfide group.

4. The polyamide of any of the preceding claims where the monomer comprising the 2-oxazolinyl-furan group is of formula II or III:

II

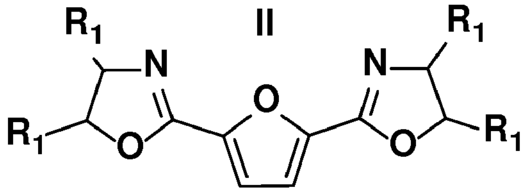

III

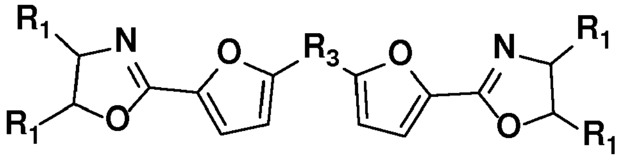

where each $R_1$ is independently of one another selected from a proton and an alkyl group and where $R_3$ is selected from a C=O group and an alkanediyl group.

**5.** The polyamide of any of the preceding claims having a crosslinking degree of at least 5%, preferably at least 10%, more preferably at least 20%.

**6.** Resin composition comprising the polyamide of claims 1 to 5.

**7.** Resin composition of claim 6 comprising reinforcements and/or fillers.

**8.** Resin composition of claim 7 wherein the reinforcements are fibrous reinforcements, preferably natural, inorganic or synthetic fibers, more preferably glass fibers, polyamide fibers, or carbon fibers.

**9.** A shaped product comprising the resin composition of claims 6 to 8.

**10.** The shaped product of claim 9, which is a coating, film, adhesive or laminate.

**11.** Process for manufacturing a polyamide from a mixture of monomers, whereby a monomer which comprises a 2-oxazolinyl-furan group is reacted with a bis-, tri or multifunctional carboxylic acid, amine, sulfide, alcohol or amide or any combinations thereof.

**12.** The process of claim 11 where 0.01 to 10 wt% of a catalyst is added to the mixture of monomers, preferably 0.1 to 5 wt%, more preferably 0.2 to 2 wt%.

**13.** The process of claim 11 or 12 where the catalyst is selected from phosphorous acid esters, organic phosphonous acids and inorganic salts or combinations thereof.

**14.** Use of monomers comprising a 2-oxazolinyl-furan group for polymerization reactions.

Fig. 1

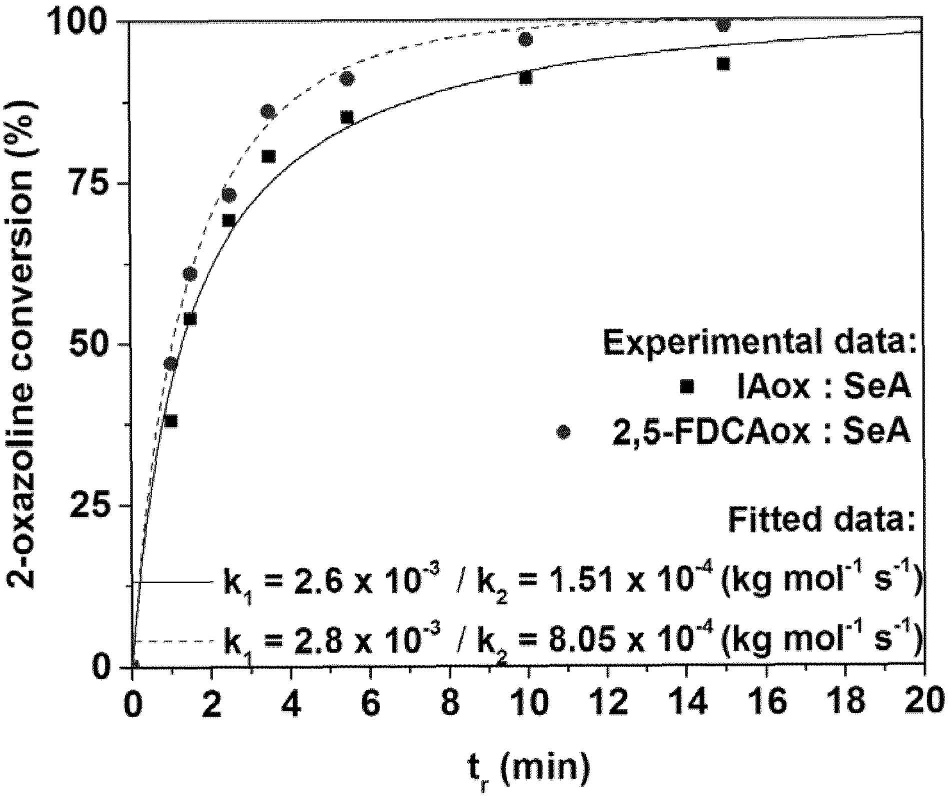
100
75
50
25
0
2-oxazoline conversion (%)
0 2 4 6 8 10 12 14 16 18 20
$t_r$ (min)
Experimental data:
IAox : SeA
2,5-FDCAox : SeA
Fitted data:
$k_1 = 2.6 \times 10^{-3}$ / $k_2 = 1.51 \times 10^{-4}$ (kg $mol^{-1}$ $s^{-1}$)
$k_1 = 2.8 \times 10^{-3}$ / $k_2 = 8.05 \times 10^{-4}$ (kg $mol^{-1}$ $s^{-1}$)

Fig. 2

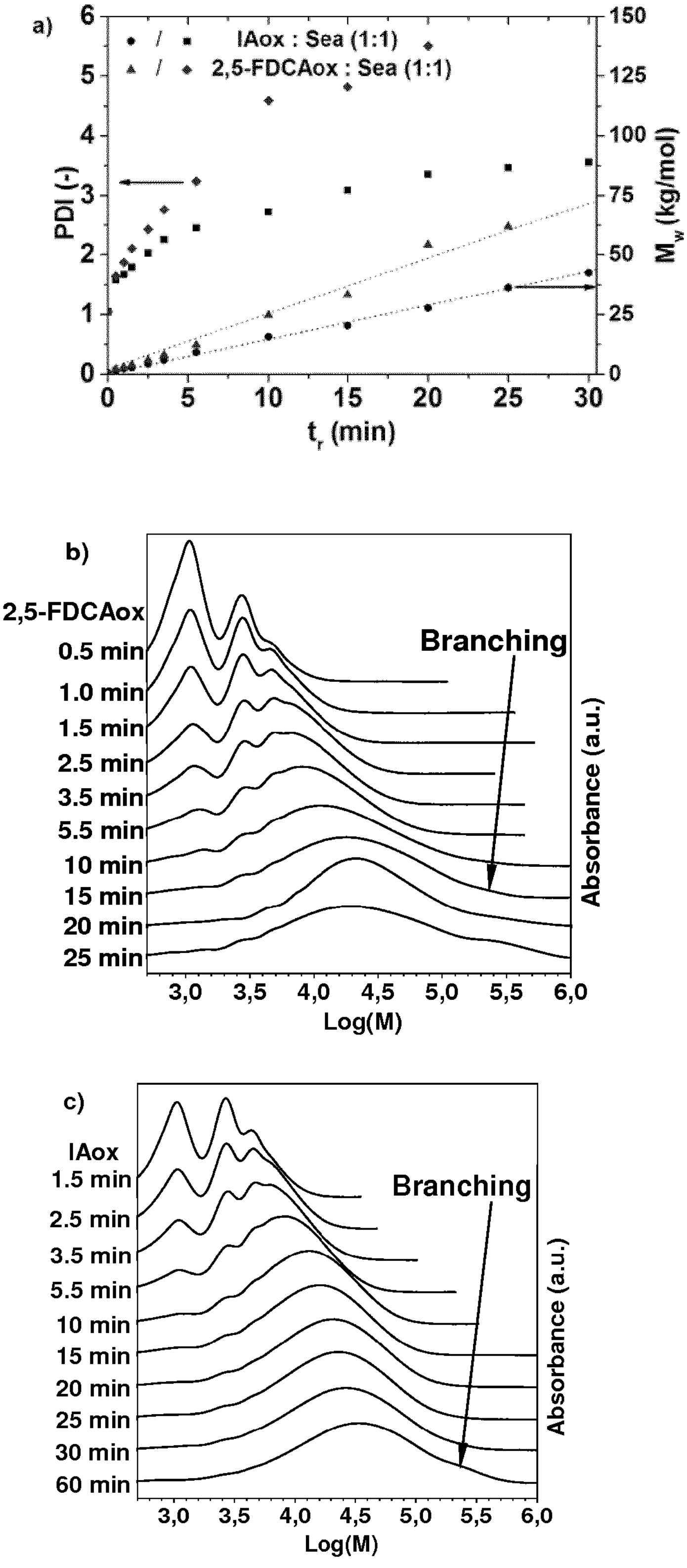
a)
IAox : Sea (1:1)
2,5-FDCAox : Sea (1:1)
PDI (-)
$M_w$ (kg/mol)
$t_r$ (min)
b)
2,5-FDCAox
0.5 min
1.0 min
1.5 min
2.5 min
3.5 min
5.5 min
10 min
15 min
20 min
25 min
Branching
Absorbance (a.u.)
Log(M)
c)
IAox
1.5 min
2.5 min
3.5 min
5.5 min
10 min
15 min
20 min
25 min
30 min
60 min
Branching
Absorbance (a.u.)
Log(M)

Fig. 3

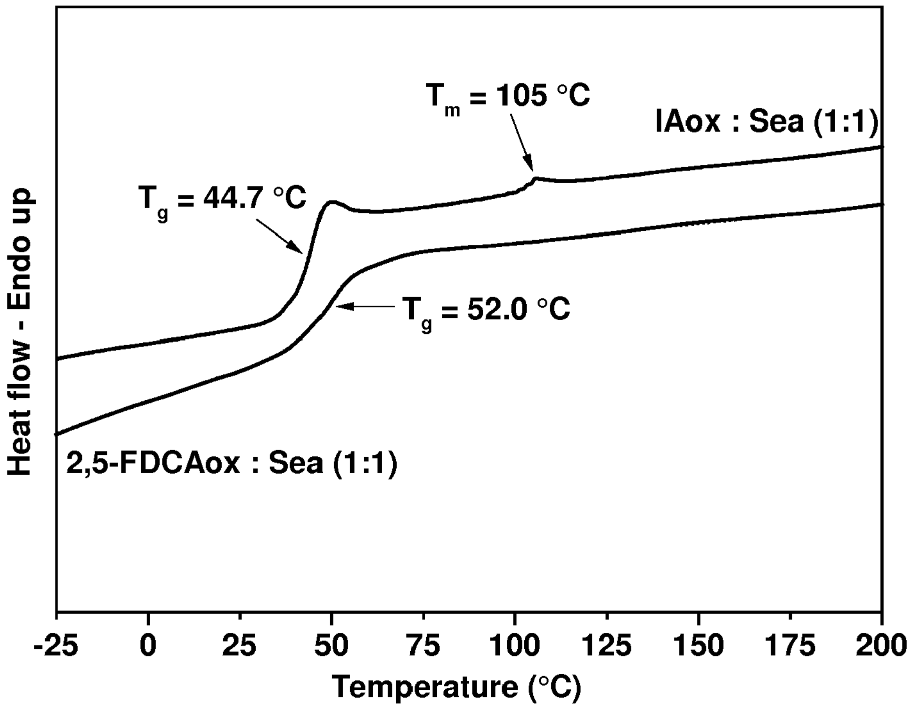

$T_m$ = 105 °C
IAox : Sea (1:1)
$T_g$ = 44.7 °C
$T_g$ = 52.0 °C
2,5-FDCAox : Sea (1:1)
Heat flow - Endo up
-25
0
25
50
75
100
125
150
175
200
Temperature (°C)

Fig. 4

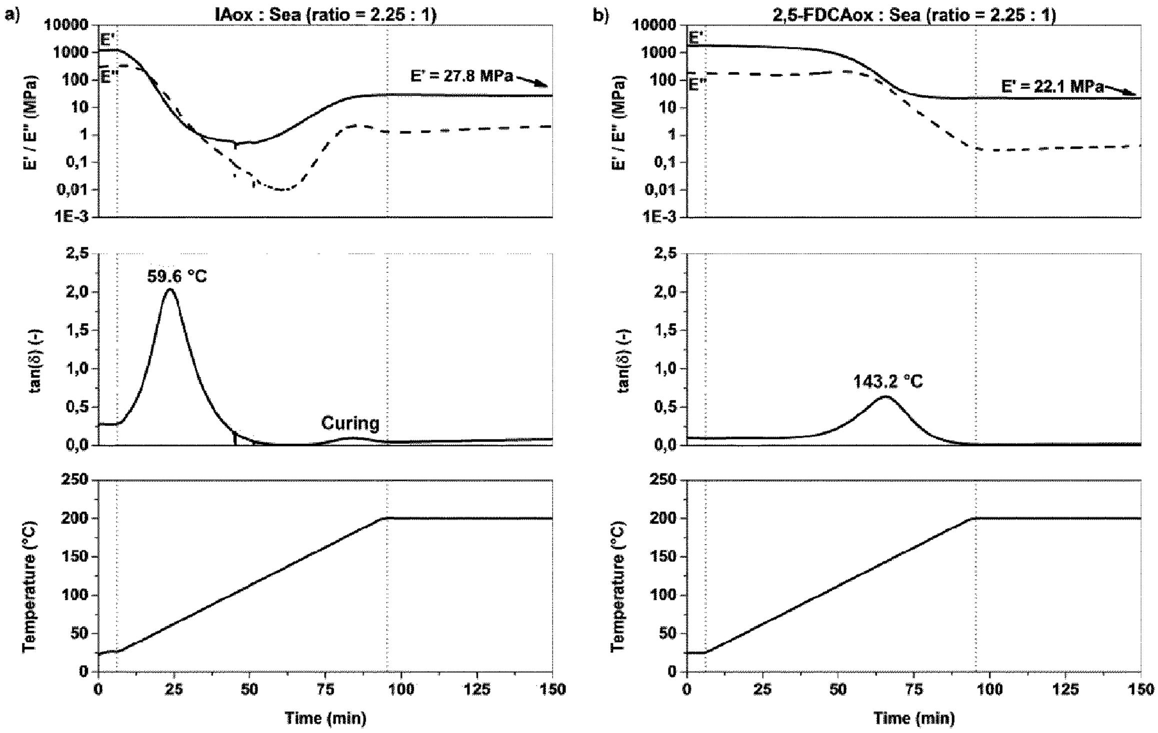
a)
IAox : Sea (ratio = 2.25 : 1)
E'
E''
E' = 27.8 MPa
E' / E'' (MPa)
59.6 °C
Curing
tan(δ) (-)
Temperature (°C)
Time (min)
b)
2,5-FDCAox : Sea (ratio = 2.25 : 1)
E' = 22.1 MPa
143.2 °C

Fig. 5

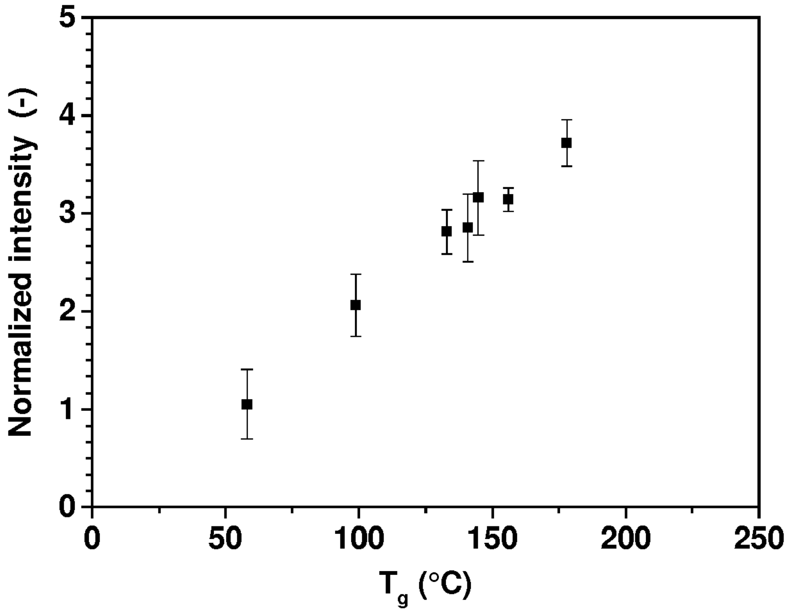

Europäisches Patentamt
European Patent Office
Office européen des brevets

# EUROPEAN SEARCH REPORT

Application Number
EP 14 19 3170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | LAURENT NÉRY ET AL: "Kinetic and Mechanistic Studies of Carboxylic Acid-Bisoxazoline Chain-Coupling Reactions", MACROMOLECULAR CHEMISTRY AND PHYSICS, vol. 204, no. 14, 1 September 2003 (2003-09-01), pages 1755-1764, XP055182950, ISSN: 1022-1352, DOI: 10.1002/macp.200350036 * the whole document * ----- | 1-14 | INV. C08G69/44 C08L77/12 C07D413/04 C07D413/14 |
| Y | WEI JIE LI ET AL: "A facile and efficient synthesis of bis(oxazoline)s", JOURNAL OF HETEROCYCLIC CHEMISTRY, vol. 47, no. 6, 23 August 2010 (2010-08-23), pages 1340-1343, XP055183124, ISSN: 0022-152X, DOI: 10.1002/jhet.477 * the whole document * ----- | 1-14 | |
| Y | DATABASE WPI Week 200740 Thomson Scientific, London, GB; AN 2007-413586 XP002738495, -& CN 1 884 283 A (UNIV SHANTOU) 27 December 2006 (2006-12-27) * abstract; claim 1 * ----- -/-- | 1-14 | TECHNICAL FIELDS SEARCHED (IPC) C08G C08L C07D |

The present search report has been drawn up for all claims

| Place of search | Date of completion of the search | Examiner |
|---|---|---|
| Munich | 17 April 2015 | Otegui Rebollo, Juan |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 3170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROSSEGGER E ET AL: "Design Strategies for Functionalized Poly(2-oxazoline)s and Derived Materials", POLYMERS MDPI AG SWITZERLAND, vol. 5, no. 3, September 2013 (2013-09), pages 956-1011, XP002738496, ISSN: 2073-4360, DOI: 10.3390/POLYM5030956 * abstract * * page 957, paragraph 1 - page 958, paragraph 3; table 1 * * page 965, paragraph 1 * * page 982, paragraph 2 - page 983, paragraph 1 * * page 995, paragraph 3 - page 996, paragraph 7 * ----- | 14 | |
| X | KEMPE K ET AL: "Synthesis and characterization of a series of diverse poly(2-oxazoline)s", JOURNAL OF POLYMER SCIENCE, PART A: POLYMER CHEMISTRY, vol. 47, no. 15, 1 August 2009 (2009-08-01), pages 3829-3838, XP002738497, JOHN WILEY AND SONS INC. USA DOI: 10.1002/POLA.23448 * abstract * * page 3831, left-hand column, last paragraph - page 3835, right-hand column, paragraph 1; table 2 * ----- | 14 | TECHNICAL FIELDS SEARCHED (IPC) |
| A,D | EP 0 097 937 A1 (TAKEDA CHEMICAL INDUSTRIES LTD [JP]) 11 January 1984 (1984-01-11) * the whole document * ----- | 1-14 | |
| | -/-- | | |

The present search report has been drawn up for all claims

2

| Place of search | Date of completion of the search | Examiner |
|---|---|---|
| Munich | 17 April 2015 | Otegui Rebollo, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 3170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | Wilsens, Carolus et al: "Synthesis, kinetics, and characterization of bio-based thermosets obtained through polymerization of a 2,5-furandicarboxylic acid-based bis(2-oxazoline) with sebacic acid",<br>Polymer Chemistry,<br>vol. 6<br>13 February 2015 (2015-02-13), pages 2707-2716, XP002738498,<br>DOI: 10.1039/c4py01609b<br>Retrieved from the Internet:<br>URL:http://pubs.rsc.org/en/content/articlepdf/2015/py/c4py01609b<br>[retrieved on 2015-04-16]<br>* the whole document *<br>----- | 1-14 | |
| | | | TECHNICAL FIELDS SEARCHED (IPC) |

The present search report has been drawn up for all claims

| Place of search | Date of completion of the search | Examiner |
|---|---|---|
| Munich | 17 April 2015 | Otegui Rebollo, Juan |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 3170

This annex lists the patent family members relating to the patent documents cited in the above-mentioned European search report. The members are as contained in the European Patent Office EDP file on
The European Patent Office is in no way liable for these particulars which are merely given for the purpose of information.

17-04-2015

| Patent document cited in search report | Publication date | Patent family member(s) | Publication date |
|---|---|---|---|
| CN 1884283 A | 27-12-2006 | NONE | |
| EP 0097937 A1 | 11-01-1984 | CA 1192346 A1 | 20-08-1985 |
| | | DE 3361216 D1 | 19-12-1985 |
| | | EP 0097937 A1 | 11-01-1984 |
| | | US 4474942 A | 02-10-1984 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4474942 A


### Non-patent literature cited in the description


- **NERY et al.** *Macromol. Chem. Phys.,* 2003, vol. 204, 1755-1764